# EUROPEAN PATENT SPECIFICATION

(11) **EP 3 710 115 B1**
(45) Date of publication and mention of the grant of the patent: **14.06.2023**
(21) Application number: 18800951.8
(22) Date of filing: 14.11.2018
(51) Int. Cl.: A61Q 5/04, A61K 8/41, A61K 8/43, A61K 8/44, A61K 8/46, A61K 8/22, A61K 8/23

(54) **PROCESS FOR PERMANENT WAVING KERATIN FIBERS**
VERFAHREN ZUM DAUERWELLEN VON KERATINFASERN
PROCÉDÉ DE DÉFORMATION PERMANENTE DE FIBRES KÉRATINIQUES

(30) Priority: 17.11.2017 EP 17202293
(43) Date of publication of application: 23.09.2020
(73) Proprietor: Kao Germany GmbH, 64297 Darmstadt Hessen (DE)
(72) Inventor: SCHNEIDER, Jörg, 64297 Darmstadt (DE); HEILMANN, Jens, 64297 Darmstadt (DE)
(74) Representative: Grit, Mustafa
(86) International application number: PCT/EP2018/081170
(87) International publication number: WO 2019/096815

(56) References cited:
- EP-A1- 0 673 640
- EP-A1- 1 880 709
- EP-A1- 2 332 515
- US-A1- 2013 276 809

## Description

Present invention relates to a process for permanent waving keratin fibers, especially human hair, for achieving durable waves.

The well-known and commonly used process for permanent waving keratin fibers involves reducing and oxidizing steps which is often perceived as fiber damaging and the results are very much dependent upon how the whole process is carried out. The fiber damage is especially due to the inappropriately selected, if not adjusted, processing period of the reducing composition. The hair may easily become over processed and appear, therefore, to have less strength, be brittle and especially not naturally feeling upon touching.

Another aspect is that the use and/or the need of heat application during processing of the reducing agent on the fibers. This is usually realized with an external electrical heating devices and, especially in Asian geography, a specially therefore designed machine so called heat perming machine is used. It is the observation of the applicant that unless the pre-reduced hair is processed with heat, almost no curls are obtained although the hair is considerably damaged. There is highly need for simplified and non-damaging processes for obtaining strong, natural feeling, homogenous permanent waving.

The EP 673 640 discloses a process for permanent waving hair wherein hair is treated with a reducing composition for a period of 20 min and, without rinsing off, an alkaline composition was applied comprising alkali carbonates and hydrogen carbonates for a period of 10 min and finally hair is applied an aqueous oxidizing composition. It has been observed that the process described therein does not deliver cosmetically acceptable hair qualities in terms of waving efficiency and especially in smoothness and softness.

Furthermore, JP-H03-153621 discloses a permanent shaping process wherein an acidic composition is mixed with an alkaline reducing agent composition and applied onto hair and after certain processing time an oxidizing composition is applied onto hair. The process does not deliver cosmetically appealing curls and hair qualities. Similar process to the above is disclosed in US2008/0142033 wherein after treating hair with reducing composition, a composition comprising monovalent cation salt of organic acids is applied and finally hair is oxidized. This process as well have drawbacks in delivering less damage and effective curling to the hair which at the same time feels soft and smooth upon touching.

EP2332515A1 discloses a process for permanent shaping of hair, where prior or during or efter application of the reducing agent, hair is put on curlers which are taken out prior to or during or after application of the oxidizing composition.

The inventors of the present invention have unexpectedly found out that application of an intermediate aqueous alkaline composition onto reduced keratin fibers, especially human hair, wherein the reducing composition is rinsed off delivers soft and smooth hair with well-defined strong curls. The hair waved with such processes feels natural upon touching, has natural appearance with homogenous and intensive bouncy curls.

Thus, the first object of the present invention is a process for permanent waving keratin fibers, especially human hair, wherein,
a- optionally, the keratin fibers especially human hair is washed and/or shampooed, and towel dried,
b- an aqueous composition comprising one or more reducing agent at a total concentration in the range of 0.5 to 20% by weight, calculated to the total of the aqueous composition, is applied and left on the fibers for a period of 1 to 60 min,
c- the fibers are rinsed off,
d- the fibers are put on curlers,
e- a non-reducing and non-oxidizing aqueous composition comprising one or more alkalizing agent and having a pH in the range of 8.5 to 12 is applied onto fibers and left on the fibers for a period 1 to 60 min,
f- optionally the fibers are rinsed off,
g- an aqueous composition comprising one or more oxidizing agents, preferably hydrogen peroxide or bromate salt, is applied onto fibers and left on the fibers for a period 1 to 30 min,
h- the fibers are optionally rinsed off, and
i- the fibers are dried,
wherein the curlers are taken off from fibers before or during processing in step g or after the step g prior to rinsing off and/or drying.

The second object of the present invention is the use of a process of the present invention for achieving natural, intensive, homogeneous waves on keratin fibers, especially human hair.

The third object of the present invention is a kit for keratin fibers, especially human hair comprising the compositions used in the process above, namely an aqueous composition comprising one or more reducing agents, a non-reducing and non-oxidizing aqueous composition comprising one or more alkalizing agents, and an aqueous composition comprising one or more oxidizing agents, preferably hydrogen peroxide or bromate salt.

In a further preferred embodiment of the present invention, in order to prevent fibers drying during processing, the fibers, especially human hair, are covered with e.g. foil or towel, especially during the periods the compositions comprising reducing agents and alkalizing agents are left on the fibers.

In a further preferred embodiment of the present invention, the whole process is carried out at ambient temperature without using any heat and/or heating device. Without being bound by the theory, this should even further be beneficial to reduce hair damage and therefore contribute to healthy appearance and feeling of the fibers.

In the process of the present invention, an aqueous composition comprising one or more reducing agents is applied onto fibers. In principle any reducing agent of inorganic and organic ones and their mixtures are suitable for the purpose of the present invention. The preferred ones are inorganic and organic reducing agents.

Suitable inorganic reducing agents are sulfite and/or hydrogen sulfite salts such as sodium, potassium, ammonium and suitable organic reducing agents are thiogylcolic acid and/or its salts, cysteamine and/or its salts, thioglycerin and/or its salts, glycerin esters of thioglycolic acid and/or its salts, thiolactic acid and/or its salts, cysteine or its derivatives and/or its salts, and their mixtures. Preferred are thioglycolic acid and/or its salts, thiolactic acid and/or its salts, cysteine or its derivatives and/or its salts and sodium, potassium, ammonium sulfites and their mixtures. The most preferred are thioglycolic acid and/or its salts and sodium, potassium, ammonium sulfites, and their mixtures.

The total concentration of reducing agents in the aqueous composition of step b is in the range of 0.5 to 20%, preferably 1 to 15%, more preferably 2 to 12% and most preferably 3 to 10% by weight calculated to the total of the aqueous composition.

The pH of the composition may be acidic or alkaline and preferably in the range of 3 to 12, more preferably 4 to 11 and most preferably it is alkaline and in the range of 7.5 to 10.5. The pH may be adjusted with the known organic and/or inorganic acids and alkalizing agents (see below).

The aqueous composition comprising one or more reducing agents is left on the hair for a period of 1 to 60 min, preferably 2 to 45 min, more preferably 5 to 30 min and most preferably 5 to 20 min at ambient temperature and without using any heat and/or heating device.

After rinsing off the fibers, the fibers are put on curlers and applied a non-reducing and non-oxidizing aqueous composition comprising one or more alkalizing agents. The composition is left on the hair for a period of 1 to 60 min, preferably 2 to 45 min, more preferably 5 to 30 min and most preferably 5 to 20 min at ambient temperature and without using any heat and/or heating device.

The suitable alkalizing agents may be inorganic and organic ones and all well-known agents are suitable for the purpose of the present process. The pH of the composition is in the range of8.5 to 12, preferably 8.5 to 10.5 and more preferably 8.5 to 10. The pH may be adjusted by selecting the concentration of the alkalizing agent for achieving the required pH or alternatively may be adjusted using inorganic and/or organic acids.

The concentration of the alkalizing agents is adjusted as their NH₃ equivalent which may easily be determined by titrating the alkaline solution with a standard acidic solution and calculating the molar concentration of the alkalizing agent which is then expressed as molar equivalent NH₃. Finally, from the molar equivalent and from the molecular weight of NH₃, the concentration in % is calculated. The titration is carried out at ambient temperature i.e. at approximately 20°C. Accordingly, the ammonia equivalent concentration must be in the range of 0.1 to 15%, preferably 0.1 to 12.5%, more preferably 0.25 to 10% and most preferably 0.5 to 8% by weight calculated to the total of the composition as ammonia equivalent determined by titration method at ambient temperature.

Suitable alkalizing agents are the alkali hydroxides such as sodium hydroxide, potassium hydroxide, ammonia and its salts such as ammonium carbonate, ammonium hydrogen carbonate, ammonium carbamate, ammonium chloride, ammonium sulfate, ammonium phosphates such as ammonium dihydrogen phopshate, diammonium hydrogen phosphate, diammonium sodium phosphate, ammonium sodium hydrogen phosphate or ammonium disodium phosphate, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate, guanidine and its salts such as guanidine hydrochloride, guanidine carbonate, guanidine bicarbonate, and an alkyl or alkanol amine according to the general structure 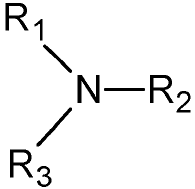 or R₃ is different from H, such as monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures.

Preferred are ammonia and its salts, monoethanolamine, diethanolamine, triethanolamine, amino methyl propanol, guanidine salts and their mixtures. The most preferred are ammonia, ammonium chloride, guanidine carbonate, monoethanolamine and amino methyl propanol and their mixtures.

After rinsing off the aqueous composition comprising alkalizing agents, an aqueous composition comprising one or more oxidizing agents, preferably hydrogen peroxide or a bromate salt is applied onto hair and left on the hair for 0.5 to 30 min, preferably 2 to 25 min, more preferably 3 to 20 min and most preferably 5 to 15 min at ambient temperature without application of any heat and/or heating device.

The fibers are preferably rinsed off at the end of the above referred processing time. Optionally the oxidizing composition may also be left on the hair, i.e. not rinsed off from hair.

The total concentration of one or more oxidizing agents, preferably hydrogen peroxide or bromate salt, in the aqueous composition is in the range of 0.1 to 15%, preferably 0.2 to 12.5%, more preferably 0.25 to 10% and most preferably 0.5 to 8% by weight calculated to the total of the aqueous composition.

In general the pH of the oxidizing composition is in the range of 2 to 8. The pH of the composition is depending on the oxidizing agent comprised in the composition. In case of hydrogen peroxide a pH in the range of 2 to 6 is suitable. In case of sodium bromate a pH of 5 to 8 is suitable. pH of the composition may be adjusted using inorganic and/or organic acids and bases well known in the art.

The curlers are being taken off from hair prior to application of the aqueous oxidizing composition or during the time the aqueous composition is left on the hair or after rinsing off the aqueous oxidizing composition. The preferred is the curlers are taken off from hair after rinsing off the aqueous oxidizing composition.

In case that the aqueous oxidizing composition is not rinsed off from hair, the curlers may be taken off from hair either after application of the oxidizing composition or prior to application of the oxidizing composition.

In the following, all reported concentrations must be understood as relative to each of the compositions because, firstly, the compositions are not mixed with each other and secondly, the same ingredient disclosed must not be comprised in all of the composition, although this is a possibility.

Aqueous compositions, all three or one or two advantageously comprise a thickening agent, preferably a thickening polymer. Suitable and preferred ones are thickening polymers such as polysaccharides such as alginate, pectinate, xanthan, hydroxypropyl xanthan or dehydroxanthan, non-ionic polysaccharides such as cellulose ethers (e.g., methylcellulose, hydroxyethylcellulose (HEC), methyl hydroxyethylcellulose (MHEC), ethyl hydroxyethylcellulose (EHEC), methyl ethyl hydroxyethylcellulose (MEHEC)), starch or dextrins. Synthetic acrylate type of thickeners may as well be comprised such as acrylate copolymers and alkyl acrylates homo or copolymers also known as associative thickeners.

The concentration of the thickening polymer is very much dependent on the type of the thickening polymer and the targeted consistency (viscosity) of the compositions. Typically, the thickening polymers are comprised in the compositions at a concentration in the range of 0.1 to 3%, preferably 0.25 to 2% by weight calculated to the total of each of the composition.

Aqueous compositions, all three or one or two can comprise one or more fatty alcohols. Suitable fatty alcohols are the ones with the chain length of 14 to 22 C atoms which may be saturated or unsaturated, linear or branched which may as well be substituted. Non-limiting examples are myristyl alcohol, cetyl alcohol, stearyl alcohol, behenyl alcohol, octyl dodecanol, cetostearyl alcohol, and their mixtures.

The total concentration of fatty alcohol is in the range from 0.5 to 15%, preferably 1 to 10% by weight, calculated to total of each of the composition.

Aqueous compositions, all three or one or two, advantageously comprise one or more surfactants. Suitable ones are selected from anionic, non-ionic, amphoteric and cationic ones.

Anionic surfactants suitable are in principle known from the cleansing compositions. These are anionic surfactants of the sulfate, sulfonate, carboxylate and alkyl phosphate type, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates. Preferred anionic surfactants are alkyl sulphate surfactants especially lauryl sulphate and its salts.

Further suitable surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics^{R}", as well as fatty alcohol ethoxylates, C₁₀-C₂₂-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also been proven suitable.

Suitable cationic surfactants are according to the general structure 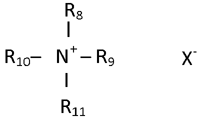 wherein R₈ is a saturated or unsaturated, branched or linear alkyl chain with 8-22 C atoms or

R₁₂ CO NH (CH₂)ₙ

where R₁₂ is saturated or unsaturated, branched or linear alkyl chain with 7-21 C atoms and n has typical value of 1 - 4 or

R₁₃ CO O (CH₂)ₙ

where R₁₃ is saturated or unsaturated, branched or linear alkyl chain with 7-21 C atoms and n has typical value of 1 - 4, and
R₉ is H or unsaturated or saturated, branched or linear alkyl chain with 1 - 22 C atoms or

R₁₂ CO NH (CH₂)ₙ

or

R₁₃ CO O (CH₂)ₙ

where R₁₂, R₁₃ and n are same as above.

R₁₀ and R₁₁ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The total concentration of one or more surfactants is in the range of 0.1 to 12.5%, preferably 0.2 to 10% and more preferably 0.5 - 7.5% by weight, calculated to the total of each of the composition.

Further advantageously, aqueous compositions, all three or one or two, comprise one or more silicone compound, preferably silicone oil. Suitable and preferred ones are known with their CTFA adopted name as dimethicone and commercially available from Dow Corning under the trade name DC 200 with various viscosities.

Further advantageously, aqueous compositions, all three or one or two, comprise one or more cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

Preferred are Polyquaternium -2, Polyquaternium -6 and Polyquaternium 16.

The total concentration of cationic polymers may be in the range of 0.1 - 2.5%, preferably 0.25 - 2% by weight and more preferably 0.5 - 1.5% by weight, calculated to total of each of the composition.

Further advantageously, aqueous compositions all three or one or two comprise one or more aminated silicones which may be selected from amodimethicones and grafted aminianted siliocnes. Suitable ones are available under various trade names such as DC 969, Belsil from Wacker Chemie AG and know with the CTFA adopted name Amodimethicone, and Elastomer OS from Kao Corporation known with CTFA adopted name Polysilicone-9.

Furthermore, aqueous compositions, all three or one or two, comprise one or more organic solvent which may act as penetration enhancer and/or solubilizing agent for the compounds not readily soluble in the aqueous medium. The suitable ones are 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol.

Concentration of one or more organic solvent is in the range of 0.1 to 15%, preferably 0.5 to 12.5% and more preferably 1 to 10% and most preferably 1 to 7.5% by weight calculated to the total of each of the composition.

The aqueous compositions, all three or one or two may advantageously comprise urea, at a concentration in the range of 0.1 to 20%, preferably 1 to 15% by weight calculated to the total of the compositions.

Additionally, the aqueous compositions, all three or one or two, comprise one or more polyols. Suitable ones are glycerine, phytantriol, panthenol, ethyleneglycol, polyethyleneglycols, propylene glycols such as 1,2 propylene glycol, 1,3-propylene glycol and polypropylene glycols.

The total concentration of one or more polyol is in the range of 0.1 to 15%, preferably 0.25 to 12.5%, more preferably 0.5 to 10% and most preferably 1 to 7.5% by weight calculated to the total of each of the composition.

The aqueous compositions, all three or one or two, can comprise one or more amino acids and/or their water soluble salts. Suitable ones are glycine, histidine, citrullin, asaparagine, alanine, valine. Leucine, isoleucine, proline, tryptophan, phenylalanine, methinone, serine, tyrosine, threonine and glutamine.

The total concentration of one or more aminoacids and/or their water soluble salts is in the range of 0.01 to 2.5%, preferably 0.1 to 2%, more preferably 0.15 to 1.5% and most preferably 0.2 to 1 % by weight calculated to the total of each of the composition.

Any of the compositions described in detail above may comprise ingredients customarily found in such compositions such as preservative, fragrance, chelating agents, radical scavenger, etc.

Following example to illustrate the invention, but not to limit it.

### Example 1

| Aqueous reducing composition | |
|---|---|
| | % by weight |
| Ammonium thioglycolate | 10 |
| Ammonium hydroxide | 2 |
| Water | to 100 |

The pH of the above composition was adjusted to pH 9.0.

| Aqueous alkaline composition | |
|---|---|
| | % by weight |
| Ammonium hydroxide | 5 |
| Water | to 100 |

The pH of the above composition was adjusted to pH 9.9.

Aqueous oxidizing composition

| | |
|---|---|
| Hydrogen peroxide | 3 |
| Phosphoric acid | q.s. to pH 3 |
| Water | to 100 |

A hair streak weighing approximately 5 g and having a length of 20 cm is permanently waved using the above compositions. Firstly the streak was washed with a commercially available shampoo compositions and towel dried. Afterwards, the streak was dipped into the aqueous reducing composition and left in the solution for 15 min and taken out and rinsed off with water. Afterwards, the streak was put on curlers with a diameter of 1.5 cm and an alkaline dipped into the alkaline composition of above. After 10 min, taken out and rinsed off and dipped into the oxidizing composition for 15 min and the curlers were taken off. It was observed that the streak was well waved.

### Example 2

| Aqueous reducing composition | |
|---|---|
| | % by weight |
| Ammonium thioglycolate | 7 |
| Ammonium bicarbonate | 4 |
| Phosphoric acid | q.s. to pH 6.5 |
| Water | to 100 |

| Aqueous alkaline composition | |
|---|---|
| | % by weight |
| Ammonium bicarbonate | 4 |
| Sodium hydroxide | q.s. to pH 8.5 |
| Water | to 100 |

| Aqueous oxidizing composition | |
|---|---|
| | % by weight |
| Sodium bromate | 5 |
| Water | to 100 |

The above composition had a pH of 7.0.

Using the above compositions, two hair streaks as in example 1 were permanently waved comparatively to the process disclosed in EP 673640.

The hair streaks were shampooed and the first reducing agents were applied and left on the streaks for 20 min, Afterwards, one of the streaks was rinsed off with water and the other was not rinsed off. Both streaks were put on curlers having a dimeter of 1.5 cm. Both streaks were soaked into the alkaline composition and left for 10 min. Subsequently, the streak not rinsed off was directly soaked into the oxidizing composition whereas the other first rinsed off and afterwards soaked into the oxidizing composition. After 15 min both were taken out from the oxidizing composition and rinsed off with water and the curlers were taken off. The streaks were dried with a hair drier.

It was observed that the streak which was rinsed off after treating with reducing agent was waved much more effectively and also felt smoother and softer upon touching. The streaks were analyzed by a panel of 7 hair dressers for their waving appearance, softness and smoothness in a scale 1 to 5 wherein 1 is not good at all and 5 is being the best performance. The following results were obtained for the streaks treated according to the present invention and comparative process according to EP673640.

| | Inventive | SD | Comparative | SD |
|---|---|---|---|---|
| Wave appearance | 3.50 | 0.40 | 2.45 | 0.38 |
| Softness | 3.71 | 0.45 | 2.52 | 0.44 |
| Smoothness | 3.85 | 0.35 | 2.42 | 0.49 |

The SD means standard deviation of the mean values.

From above results it is beyond any doubt that the process according to the process of the present invention delivers very much improved permanent waved hair. Since there is only one difference which is the rinsing off steps between the applications of various compositions, the improved results are clearly attributed to this.

### Example 3

| Aqueous reducing composition | |
|---|---|
| | % by weight |
| Ammonium thioglycolate | 7 |
| Ammonium bicarbonate | 4 |
| Phosphoric acid | q.s. to pH 6.5 |
| Water | to 100 |

| Aqueous alkaline composition | |
|---|---|
| | % by weight |
| Ammonium bicarbonate | 4 |
| Sodium hydroxide | q.s. to pH 8.5 |
| Water | to 100 |

| Aqueous oxidizing composition | |
|---|---|
| | % by weight |
| Sodium bromate | 5 |
| Water | to 100 |

The above composition had a pH of 7.0.

Using the above compositions, two hair streaks as in example 1 were permanently waved comparatively to the process disclosed in EP 673640.

The hair streaks were shampooed and the first reducing agents were applied and left on the streaks for 20 min, Afterwards, one of the streaks was rinsed off with water and the other was not rinsed off. Both streaks were put on curlers having a diameter of 1.5 cm. Both streaks were soaked into the alkaline composition and left for 10 min. Subsequently, both streaks were directly soaked into the oxidizing composition and after 15 min both were taken out from the oxidizing composition and rinsed off with water and the curlers were taken off. The streaks were dried with a hair drier.

It was observed that the streak which was rinsed off after treating with reducing agent was waved much more effectively and also felt smoother and softer upon touching. The streaks were analyzed by a panel of 7 hair dressers for their waving appearance, softness and smoothness in a scale 1 to 5 wherein 1 is not good at all and 5 is being the best performance. The following results were obtained for the streaks treated according to the present invention and comparative process according to EP673640.

| | Inventive | SD | Comparative | SD |
|---|---|---|---|---|
| Wave appearance | 3.65 | 0.41 | 2.23 | 0.31 |
| Softness | 3.77 | 0.43 | 2.41 | 0.38 |
| Smoothness | 3.95 | 0.33 | 2.23 | 0.34 |

The SD means standard deviation of the mean values.

From above results it is beyond any doubt that the process according to the process of the present invention delivers very much improved permanent waved hair. Since there is only one difference which is the rinsing off steps prior to the application of alkaline composition, the improved results are clearly attributed to this difference.

The following compositions are used in the process of the present invention.

### Example 4

### Aqueous reducing composition

| | % by weight | |
|---|---|---|
| Cetearyl alcohol | | 10 |
| Oleyl alcohol | | 5 |
| Thioglycolic acid | | 1.7 |
| Ceteareth-20 | | 1.0 |
| Hydroxyethylcellulose | | 0.5 |
| EDTA | | 0.5 |
| Fragrance | | 0.5 |
| Aminomethylpropanol | | q.s. to pH 9.0 |
| Water | | to 100 |

| Aqueous alkaline aerosol foam composition | | |
|---|---|---|
| | % by weight | |
| Ammonium hydroxide | | 5 |
| Ammonium chloride | | 2 |
| Monoethanolamine | | q.s. 9.0 |
| Cetrimonium chloride | | 2 |
| Hydroxyethylcellulose | | 0.5 |
| Water | | to 100 |

The composition was filled in an aerosol can with 90% the above composition and 10% propane butane mixture as the propellant. The can was equipped with a foam dispensing actuator and head.

| Aqueous oxidizing composition | | |
|---|---|---|
| | % by weight | |
| Cetearyl alcohol | | 5 |
| Behenrimonium chloride | | 2 |
| Hydrogen peroxide | | 3 |
| Phosphoric acid | | to pH 3.0 |
| Water | | to 100 |

The above composition was applied in the same way of the inventive process of the previous examples. The hair streak was shampooed and the first reducing agents were applied and left on the streaks for 20 min, Afterwards, the streak was rinsed off with water and was put on curlers having a dimeter of 2.0 cm. The streak was soaked into the alkaline composition and left for 10 min. Subsequently, the streak was rinsed off with water and was soaked into the oxidizing composition and after 15 min. it was taken out from the oxidizing composition and rinsed off with water and the curlers was taken off. The streak was dried with a hair drier. It was observed that the streak was effectively and homogeneously curled and felt soft and smooth upon touching.

## Claims

1. A process for permanently waving keratin fibers, especially human hair, wherein,
a- optionally, the fibers are washed and/or shampooed, and towel dried,
b- an aqueous composition comprising one or more reducing agent at a total concentration in the range of 0.5 to 20%, by weight, calculated to the total of the aqueous composition, is applied and left on the fibers for a period of 1 to 60 min,
c- the fibers are rinsed off,
d- the fibers are put on curlers,
e- a non-reducing and non-oxidizing aqueous composition comprising one or more alkalizing agent and having a pH in the range of 8.5 to 12 is applied onto fibers and left on the fibers for a period 1 to 60 min,
f- optionally the fibers are rinsed off,
g- an aqueous composition comprising one or more oxidizing agents, preferably hydrogen peroxide or bromate salt, is applied onto fibers and left on the fibers for a period 1 to 30 min,
h- the fibers are optionally rinsed off, and
i- the fibers are dried,
wherein the curlers are taken off from fibers before or during processing in step g or after the step g prior to rinsing off and/or drying

2. The process according to claim 1 wherein the fibers are covered, preferably with a foil or towel, during the period the aqueous compositions comprising reducing agents and alkalizing agents are left on the fibers.

3. The process according to claims 1 and/or 2 wherein one or more reducing agents are selected from sulfite and/or hydrogen sulfite salts, thiogylcolic acid and/or its salts, cysteamine and/or its salts, thioglycerin and/or its salts, glycerin esters of thioglycolic acid and/or its salts, thiolactic acid and/or its salts, cysteine or its derivatives and/or its salts, and their mixtures.

4. The process according to any of the claims 1 to 3 wherein the total concentration of the reducing agents in the aqueous composition of step b is in the range of 1 to 15%, by weight, calculated to the total of the aqueous composition.

5. The process according to any of the claims 1 to 4 wherein the aqueous composition of step b has a pH in the range of 3 to 12.

6. The process according to any of the claims 1 to 5 wherein the process as a whole is carried out at ambient temperature without using any heat and/or heating device.

7. The process according to any of the preceding claims wherein the pH of the non-reducing and non-oxidizing aqueous composition in step e is in the range of 8.5 to 10.5 and preferably 8.5 to 10.

8. The process according to any of the preceding claims wherein the composition in step e comprises alkalizing agent at an ammonia equivalent concentration of 0.1 to 15% by weight calculated to the total of the composition as ammonia equivalent determined by titration method.

9. The process according to any of the preceding claims wherein the composition in step e comprises one or more alkalizing agents selected form alkali hydroxides such as sodium hydroxide, potassium hydroxide, ammonia and its salts such as ammonium carbonate, ammonium hydrogen carbonate, ammonium carbamate, ammonium chloride, ammonium sulfate, ammonium phosphates such as ammonium dihydrogen phopshate, diammonium hydrogen phosphate, diammonium sodium phosphate, ammonium sodium hydrogen phosphate or ammonium disodium phosphate, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate, guanidine and its salts such as guanidine hydrochloride, guanidine carbonate, guanidine bicarbonate, and an alkyl or alkanol amine according to the general structure 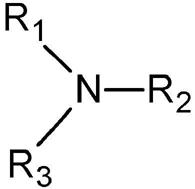 wherein R₁, R₂, and R₃ are same or different H, from C₁ to C₄, C₃ to C₄ unsaturated alkyl, C₃ to C₄ branched alkyl, C₁ to C₄ hydroxyl alkyl, C₃ to C₄ unsaturated hydroxyl alkyl, C₃ to C₄ branched hydroxyl alkyl, with the condition that at least one of R₁, R₂, or R₃ is different from H, such as monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures.

10. The process according to any of the preceding claims wherein the alkalizing agent is selected from ammonia, amino methyl propanol, monoethanolamine, sodium hydroxide, potassium hydroxide, diethanolamine, thriethanolamine, and mixtures thereof.

11. The process according to any of the preceding claims wherein the pH of the composition in step g is in the range of 2 to 8, preferably 2 to 6 when hydrogen peroxide is comprised as oxidizing agent and in the range of 5 to 8 when bromate salt is comprised in the composition.

12. The process according to any of the preceding claims wherein hydrogen peroxide or bromate salt in the aqueous composition of step g comprised at a concentration in the range of 0.1 to 10% by weight, calculated to the total of the aqueous composition.

13. The process according to any of the preceding claims wherein the curler are being taken off from the fibers after rinsing off the aqueous composition of step g and prior to drying the fibers.

14. The process according to any of the preceding claims wherein the aqueous compositions, all three or one or two of them comprise a thickening agent, preferably a thickening polymer or one or more surfactants selected from anionic, nonionic, amphoteric and cationic ones.

15. Kit for keratin fibers, especially human hair, comprising the three compositions wherein the first is an aqueous composition comprising one or more reducing agents according to claims 1 to 14, a non-reducing and non-oxidizing aqueous composition comprising one or more alkalizing agents according to claims 1 to 14 and an aqueous composition comprising one or more oxidizing agents, preferably hydrogen peroxide or a bromate salt, according to claims 1 to 14.

## Patentansprüche

1. Verfahren zur dauerhaften Verformung von Keratinfasern, insbesondere menschlichen Haaren, bei dem,
a- gegebenenfalls die Fasern gewaschen und/oder shampooniert und handtuchgetrocknet werden,
b- eine wässrige Zusammensetzung, die ein oder mehrere Reduktionsmittel in einer Gesamtkonzentration im Bereich von 0,5 bis 20 Gew.-%, berechnet auf die Gesamtmenge der wässrigen Zusammensetzung, enthält, aufgetragen und für einen Zeitraum von 1 bis 60 min auf den Fasern belassen wird,
c- die Fasern werden abgespült,
d- die Fasern werden auf Lockenwickler gelegt,
e- eine nicht-reduzierende und nicht-oxidierende wässrige Zusammensetzung, die ein oder mehrere Alkalisierungsmittel umfasst und einen pH-Wert im Bereich von 8,5 bis 12 aufweist, auf die Fasern aufgetragen wird und für einen Zeitraum von 1 bis 60 Minuten auf den Fasern verbleibt,
f- gegebenenfalls werden die Fasern abgespült,
g- eine wässrige Zusammensetzung, die ein oder mehrere Oxidationsmittel, vorzugsweise Wasserstoffperoxid oder Bromatsalz, enthält, auf die Fasern aufgebracht wird und für einen Zeitraum von 1 bis 30 Minuten auf den Fasern verbleibt,
h- die Fasern werden gegebenenfalls abgespült, und
i- die Fasern getrocknet werden,
wobei die Lockenwickler vor oder während der Verarbeitung in Schritt g oder nach dem Schritt g vor dem Abspülen und/oder Trocknen von den Fasern abgenommen werden.

2. Verfahren nach Anspruch 1, bei dem die Fasern während des Verbleibs der wässrigen Zusammensetzungen, die Reduktionsmittel und Alkalisierungsmittel enthalten, auf den Fasern abgedeckt werden, vorzugsweise mit einer Folie oder einem Handtuch.

3. Verfahren nach Anspruch 1 und/oder 2, wobei ein oder mehrere Reduktionsmittel ausgewählt sind aus Sulfit und/oder Hydrogensulfitsalzen, Thiogylkolsäure und/oder ihren Salzen, Cysteamin und/oder ihren Salzen, Thioglycerin und/oder ihren Salzen, Glycerinestern der Thioglykolsäure und/oder ihren Salzen, Thiomilchsäure und/oder ihren Salzen, Cystein oder seinen Derivaten und/oder seinen Salzen und ihren Mischungen.

4. Verfahren nach einem der Ansprüche 1 bis 3, wobei die Gesamtkonzentration der Reduktionsmittel in der wässrigen Zusammensetzung von Schritt b im Bereich von 1 bis 15 Gew.-%, berechnet auf die gesamte wässrige Zusammensetzung, liegt.

5. Verfahren nach einem der Ansprüche 1 bis 4, wobei die wässrige Zusammensetzung von Schritt b einen pH-Wert im Bereich von 3 bis 12 aufweist.

6. Verfahren nach einem der Ansprüche 1 bis 5, bei dem das gesamte Verfahren bei Umgebungstemperatur ohne Verwendung einer Wärme- und/oder Heizvorrichtung durchgeführt wird.

7. Verfahren nach einem der vorhergehenden Ansprüche, wobei der pH-Wert der nicht reduzierenden und nicht oxidierenden wässrigen Zusammensetzung in Schritt e im Bereich von 8,5 bis 10,5 und vorzugsweise 8,5 bis 10 liegt.

8. Verfahren nach einem der vorhergehenden Ansprüche, wobei die Zusammensetzung in Schritt e ein Alkalisierungsmittel in einer Ammoniakäquivalentkonzentration von 0,1 bis 15 Gew.-%, berechnet auf die Gesamtzusammensetzung als Ammoniakäquivalent, bestimmt durch ein Titrationsverfahren, umfasst.

9. Verfahren nach einem der vorhergehenden Ansprüche, wobei die Zusammensetzung in Schritt e ein oder mehrere Alkalisierungsmittel umfasst, die aus Alkalihydroxiden wie Natriumhydroxid, Kaliumhydroxid, Ammoniak und seinen Salzen wie Ammoniumcarbonat, Ammoniumhydrogencarbonat, Ammoniumcarbamat, Ammoniumchlorid, Ammoniumsulfat, Ammoniumphosphaten wie Ammoniumdihydrogenphosphat, Diammoniumhydrogenphosphat, Diammoniumnatriumphosphat, Ammoniumnatriumhydrogenphosphat oder Ammoniumdinatriumphosphat, Ammoniumnitrat, Ammoniumbromid, Ammoniumjodid, Ammoniumthiosulfat, Ammoniummolybdat, Ammoniumvanadat, Ammoniumsulfamat, Ammoniumcitrat, Ammoniumsalicylat, Ammoniumvalerat, Ammoniumtartrat, Ammoniumbenzoat, Ammoniumacetat, Ammoniumformiat und Ammoniumlactat, Guanidin und seine Salze, wie Guanidinhydrochlorid, Guanidincarbonat, Guanidinbicarbonat, und ein Alkyl- oder Alkanolamin gemäß der allgemeinen Struktur 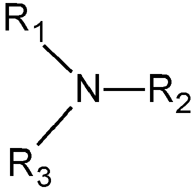 worin R₁, R₂ und R₃ gleich oder verschieden sind von H, von C₁ bis C₄, ungesättigtem C₃ bis C₄-Alkyl, verzweigtem C₃ bis C₄-Alkyl, C₁ bis C₄-Hydroxylalkyl, ungesättigtem C₃ bis C₄-Hydroxylalkyl, verzweigtem C₃ bis C₄-Hydroxylalkyl, mit der Bedingung, dass mindestens eines von R₁, R₂ oder R₃ von H verschieden ist, wie Monoethanolamin, Diethanolamin, Triethanolamin, Monoethanolmethylamin, Monoethanoldimethylamin, Diethanolmethylamin, Monoethanolethylamin, Monoethanoldiethylamin, Diethanolethylamin, Monoethanolpropylamin, Monoethanoldipropylamin, Diethanolpropylamin, Monoethanolbutylamin, Diethanolbutylamin und Aminomethylpropanol und deren Mischungen.

10. Verfahren nach einem der vorhergehenden Ansprüche, wobei das Alkalisierungsmittel ausgewählt ist aus Ammoniak, Aminomethylpropanol, Monoethanolamin, Natriumhydroxid, Kaliumhydroxid, Diethanolamin, Thriethanolamin und Mischungen davon.

11. Verfahren nach einem der vorhergehenden Ansprüche, wobei der pH-Wert der Zusammensetzung in Schritt g im Bereich von 2 bis 8 liegt, vorzugsweise 2 bis 6, wenn Wasserstoffperoxid als Oxidationsmittel enthalten ist, und im Bereich von 5 bis 8, wenn Bromatsalz in der Zusammensetzung enthalten ist.

12. Verfahren nach einem der vorhergehenden Ansprüche, wobei Wasserstoffperoxid oder Bromatsalz in der wässrigen Zusammensetzung von Schritt g in einer Konzentration im Bereich von 0,1 bis 10 Gew.-%, berechnet auf die gesamte wässrige Zusammensetzung, enthalten ist.

13. Verfahren nach einem der vorhergehenden Ansprüche, wobei die Lockenwickler nach dem Abspülen der wässrigen Zusammensetzung aus Schritt g und vor dem Trocknen der Fasern von den Fasern entfernt werden.

14. Verfahren nach einem der vorhergehenden Ansprüche, wobei die wässrigen Zusammensetzungen, alle drei oder eine oder zwei von ihnen ein Verdickungsmittel, vorzugsweise ein verdickendes Polymer oder ein oder mehrere Tenside, ausgewählt aus anionischen, nichtionischen, amphoteren und kationischen, enthalten.

15. Kit für Keratinfasern, insbesondere menschliches Haar, umfassend die drei Zusammensetzungen, wobei die erste eine wässrige Zusammensetzung ist, die ein oder mehrere Reduktionsmittel gemäß den Ansprüchen 1 bis 14, eine nicht reduzierende und nicht oxidierende wässrige Zusammensetzung, die ein oder mehrere Alkalisierungsmittel gemäß den Ansprüchen 1 bis 14 und eine wässrige Zusammensetzung, die ein oder mehrere Oxidationsmittel, vorzugsweise Wasserstoffperoxid oder ein Bromatsalz, gemäß den Ansprüchen 1 bis 14 umfasst.

## Revendications

1. Procédé d'ondulation permanente de fibres kératiniques, notamment de cheveux humains, dans lequel,
a- éventuellement, les fibres sont lavées et/ou shampooinées, et séchées à la serviette,
b- une composition aqueuse comprenant un ou plusieurs agents réducteurs à une concentration totale comprise entre 0,5 et 20%, en poids, calculée par rapport au total de la composition aqueuse, est appliquée et laissée sur les fibres pendant une période de 1 à 60 min,
c- les fibres sont rincées,
d- les fibres sont placées sur des bigoudis,
e- une composition aqueuse non réductrice et non oxydante comprenant un ou plusieurs agents alcalinisants et ayant un pH compris entre 8,5 et 12 est appliquée sur les fibres et laissée sur les fibres pendant une période de 1 à 60 minutes,
f- éventuellement, les fibres sont rincées,
g- une composition aqueuse comprenant un ou plusieurs agents oxydants, de préférence du peroxyde d'hydrogène ou du sel de bromate, est appliquée sur les fibres et laissée sur les fibres pendant une période de 1 à 30 min,
h- les fibres sont éventuellement rincées, et
i- les fibres sont séchées,
dans lequel les bigoudis sont retirés des fibres avant ou pendant le traitement à l'étape g ou après l'étape g avant le rinçage et/ou le séchage.

2. Procédé selon la revendication 1 dans lequel les fibres sont couvertes, de préférence avec une feuille ou une serviette, pendant la période où les compositions aqueuses comprenant des agents réducteurs et des agents alcalinisants sont laissées sur les fibres.

3. Procédé selon les revendications 1 et/ou 2 dans lequel un ou plusieurs agents réducteurs sont choisis parmi les sels de sulfite et/ou d'hydrogénosulfite, l'acide thiogylcolique et/ou ses sels, la cystéamine et/ou ses sels, la thioglycérine et/ou ses sels, les esters glycériques de l'acide thioglycolique et/ou ses sels, l'acide thiolactique et/ou ses sels, la cystéine ou ses dérivés et/ou ses sels, et leurs mélanges.

4. Procédé selon l'une quelconque des revendications 1 à 3, dans lequel la concentration totale des agents réducteurs dans la composition aqueuse de l'étape b est comprise entre 1 et 15%, en poids, par rapport au total de la composition aqueuse.

5. Le procédé selon l'une des revendications 1 à 4 dans lequel la composition aqueuse de l'étape b a un pH compris entre 3 et 12.

6. Le procédé selon l'une quelconque des revendications 1 à 5 dans lequel l'ensemble du procédé est réalisé à température ambiante sans utiliser de dispositif de chauffage.

7. Le procédé selon l'une quelconque des revendications précédentes dans lequel le pH de la composition aqueuse non réductrice et non oxydante à l'étape e est compris entre 8,5 et 10,5 et de préférence entre 8,5 et 10.

8. Le procédé selon l'une quelconque des revendications précédentes dans lequel la composition de l'étape e comprend un agent alcalinisant à une concentration en équivalent ammoniac de 0,1 à 15% en poids calculé par rapport au total de la composition en équivalent ammoniac déterminé par la méthode de titrage.

9. Le procédé selon l'une quelconque des revendications précédentes dans lequel la composition à l'étape e comprend un ou plusieurs agents alcalinisants choisis parmi les hydroxydes alcalins tels que l'hydroxyde de sodium, l'hydroxyde de potassium, l'ammoniac et ses sels tels que le carbonate d'ammonium, l'hydrogénocarbonate d'ammonium, le carbamate d'ammonium, le chlorure d'ammonium, le sulfate d'ammonium, les phosphates d'ammonium tels que le dihydrogénophosphate d'ammonium, l'hydrogénophosphate de diammonium, le phosphate de sodium de diammonium, l'hydrogénophosphate de sodium d'ammonium ou le phosphate de sodium d'ammonium, l'hydrogénophosphate d'ammonium et de sodium ou le phosphate disodique d'ammonium, le nitrate d'ammonium, le bromure d'ammonium, l'iodure d'ammonium, le thiosulfate d'ammonium, le molybdate d'ammonium, le vanadate d'ammonium, le sulfamate d'ammonium, le citrate d'ammonium, le salicylate d'ammonium, le valérate d'ammonium, tartarate d'ammonium, benzoate d'ammonium, acétate d'ammonium, formiate d'ammonium et lactate d'ammonium, guanidine et ses sels tels que le chlorhydrate de guanidine, le carbonate de guanidine, le bicarbonate de guanidine, et une amine d'alkyle ou d'alcanol selon la structure générale suivante 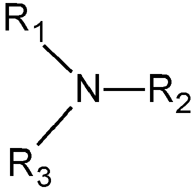 dans laquelle R₁, R₂ et R₃ sont identiques ou différents de H, de C₁ à C₄, d'alkyle insaturé de C₃ à C₄, d'alkyle ramifié de C₃ à C₄, d'alkyle hydroxyle de C₁ à C₄, d'alkyle hydroxyle insaturé de C₃ à C₄, d'alkyle hydroxyle ramifié de C₃ à C₄, à condition qu'au moins un des R₁, R₂ ou R₃ soit différent de H, comme la monoéthanolamine, la diéthanolamine, triéthanolamine, monoéthanol méthylamine, monoéthanololdiméthylamine, di-éthanolméthylamine, monoéthanolethylamine, monoéthanoldiéthylamine, diéthanolethylamine, monoéthanolpropylamine, monoéthanoldipropylamine, diéthanolpropylamine, monoéthanolbutylamine, diéthanolbutylamine et amino-méthylpropanol et leurs mélanges.

10. Le procédé selon l'une quelconque des revendications précédentes dans lequel l'agent alcalinisant est choisi parmi l'ammoniac, l'amino-méthylpropanol, la monoéthanolamine, l'hydroxyde de sodium, l'hydroxyde de potassium, la diéthanolamine, la thriéthanolamine et leurs mélanges.

11. Le procédé selon l'une quelconque des revendications précédentes dans lequel le pH de la composition à l'étape g est compris entre 2 et 8, de préférence entre 2 et 6 lorsque le peroxyde d'hydrogène est utilisé comme agent oxydant et entre 5 et 8 lorsque le sel de bromate est utilisé dans la composition.

12. Le procédé selon l'une quelconque des revendications précédentes dans lequel le peroxyde d'hydrogène ou le sel de bromate dans la composition aqueuse de l'étape g est compris dans une concentration allant de 0,1 à 10 % en poids, calculée par rapport au total de la composition aqueuse.

13. Le procédé selon l'une quelconque des revendications précédentes dans lequel les bigoudis sont retirés des fibres après rinçage de la composition aqueuse de l'étape g et avant séchage des fibres.

14. Procédé selon l'une quelconque des revendications précédentes dans lequel les compositions aqueuses, toutes les trois ou une ou deux d'entre elles comprennent un agent épaississant, de préférence un polymère épaississant ou un ou plusieurs tensioactifs choisis parmi les tensioactifs anioniques, non ioniques, amphotères et cationiques.

15. Kit pour fibres kératiniques, notamment cheveux humains, comprenant les trois compositions dont la première est une composition aqueuse comprenant un ou plusieurs agents réducteurs selon les revendications 1 à 14, une composition aqueuse non réductrice et non oxydante comprenant un ou plusieurs agents alcalinisants selon les revendications 1 à 14 et une composition aqueuse comprenant un ou plusieurs agents oxydants, de préférence du peroxyde d'hydrogène ou un sel de bromate, selon les revendications 1 à 14.
